# EUROPÄISCHE PATENTSCHRIFT

(11) **EP 2 544 636 B1**
(45) Veröffentlichungstag und Bekanntmachung des Hinweises auf die Patenterteilung: **08.04.2015**
(21) Anmeldenummer: 11712189.7
(22) Anmeldetag: 22.02.2011
(51) Int. Cl.: A61F 5/01

(54) **PALMARE DAUMEN- UND DAUMENSATTELGELENK-SCHIENE**
PALMAR THUMB AND THUMB SADDLE JOINT SPLINT
ATTELLE PALMAIRE DU POUCE ET DE L'ARTICULATION TRAPÉZO-MÉTACARPIENNE DU POUCE

(30) Priorität: 08.03.2010 CH 302102010
(43) Veröffentlichungstag der Anmeldung: 16.01.2013
(73) Patentinhaber: Chrisofix AG, 8212 Neuhausen am Rheinfall (CH)
(72) Erfinder: BOLLA, Kalman, CH-8212 Neuhausen am Rheinfall (CH)
(74) Vertreter: Rentsch Partner AG
(86) Internationale Anmeldenummer: PCT/EP2011/052603
(87) Internationale Veröffentlichungsnummer: WO 2011/110420

(56) Entgegenhaltungen:
- WO-A1-96/27349
- WO-A1-03/017887
- DE-A1- 3 519 493
- US-A- 6 146 347
- US-B1- 6 702 772

## Beschreibung

Die vorliegende Erfindung bezieht sich auf das Gebiet der medizinischen Schienen. Sie betrifft eine palmare Daumen und Daumensattelgelenk-Schiene gemäss dem Oberbegriff des Anspruchs 1.

Der Daumen der menschlichen Hand hat eine hohe Beweglichkeit und einen weiten Bewegungsbereich, um im Zusammenwirken mit den übrigen Fingern der Hand komplizierte Greifbewegungen ausführen zu können. Wesentlichen Anteil an dieser Beweglichkeit haben die verschiedenen, in ihrer Ausbildung besonderen Gelenke des Daumens. Eine zentrale Rolle spielt dabei das sogenannte "Daumen-Sattelgelenk" (Karpometakarpalgelenk) zwischen dem zur Handwurzel gehörenden grossen Vieleckbein (os trapezium) und dem ersten Mittelhandknochen (os metacarpi). Beide Knochen tragen ein wie ein Sattel geformtes Gelenkende, wobei die Gelenkflächen der Knochen quer zueinander angeordnet sind. Durch diese Anordnung sind im Daumen-Sattelgelenk Bewegungen um zwei senkrecht zueinander orientierte Hauptachsen möglich.

Eine dieser Bewegungen, die in einer Ebene schräg zur Handebene verläuft, wird als Opposition bzw. Reposition bezeichnet. In Opposition steht der Daumen den anderen Fingern zur Bildung der Greifzange gegenüber. Reponiert liegt er mit ihnen in einer Ebene. Die andere Bewegung umfasst die Abduktion und die Adduktion des Daumens. Abduktion bezeichnet das Abspreizen des Metakarpalabschnitts samt Daumen, Adduktion deren Anlegen an die Hand bei Re- oder Opposition. Wenn der Daumen radialwärts vom Zeigefinger abgespreizt ist, nimmt er Repositions- und zugleich Abduktionsstellung ein. Steht er den anderen Fingern palmar abgespreizt gegenüber (offene Greifzange), ist er im Sattelgelenk abduziert und opponiert. Die meisten Daumenbewegungen sind solche Kombinationsbewegungen um beide Achsen des Sattelgelenkes. So findet beim Daumenkreisen (Zirkumduktion) eine Kombinatifreies Ende und muss daher sehr stabil ausgebildet werden, um die notwendige Fixierung zu ermöglichen. Durch die Zunge wird eine Adduktion des Daumens verhindert, nicht jedoch eine Abduktion und insbesondere nicht eine Bewegung zwischen Opposition und Reposition. Die Zunge ist darüber hinaus unbequem, da sie mit den scharfen Seitenkanten in Daumen und Hand einschneidet, wenn der Daumen in Adduktionsrichtung bewegt wird.

Andere Daumenschienen, wie sie beispielsweise aus der US 6,520,925 oder der US 5,746,707 bekannt sind, fixieren den Daumen nur auf einer flachen Unterlage, was für die Schienung des Metacarpalbereiches in der Regel nicht ausreichend ist. Dasselbe gilt auch für Schienen der in der WO 2007/066367 gezeigten Art.

Die Druckschrift DE 35 19 493 A offenbart eine Schiene zum Ruhigstellen des Daumen-Sattelgelenkes und des Daumen-Grundgelenkes, bestehend aus einem festen Formkörper, der den Daumen und die Hand zumindest teilweise umfasst, wobei der Formkörper aus einer die Hand umschliessenden Spange, deren Enden sich in dem Daumen abgekehrten Bereichen der Hand überlappen und hier an der Überlappungsstelle miteinander durch ein Befestigungsmittel, vorzugsweise einen Klettverschluss, miteinander verbindbar sind, oder wobei der Formkörper aus einer C-förmigen Spange, die den Daumen, den Handballen, den Handrücken und die Handinnenfläche umfasst, und die über der dem Daumen abgekehrten Schmalflache der Hand mit einem Spannband geschlossen wird, aus einer an die Spange unmittelbar anschliessenden Halbschale an der Aussenseite des Daumens, die mit einem weiteren Spannband geschlossen wird, und aus einer an der Übergangsstelle Spange-Halb schale herausgeformten, in den Bereich zwischen Daumen und Handfläche hineinragenden Zunge besteht. Der Formkörper ist dabei aus Kunststoff einstückig hergestellt, wodurch der Tragekomfort erheblich eingeschränkt ist.

Es ist daher Aufgabe der vorliegenden Erfindung, eine palmare Daumen- und Daumensattelgelenk-Schiene zu schaffen, die eine spezifische und vollständige Immobilisierung des Daumen-Sattelgelenks ermöglicht, einfach aufgebaut ist, sich leicht und ohne spezielle Hilfsmittel anlegen lässt und sich vor allem durch einen hohen Tragekomfort auszeichnet. Insbesondere soll die palmare Daumen- und Daumensattelgelenk-Schiene bei der Behandlung der Rhizarthrose einsetzbar sein und die schmerzhaften Bewegungen der von der Arthrose beeinträchtigten Gelenke sicher unterbinden.

Die Aufgabe wird durch die Gesamtheit der Merkmale des Anspruchs 1 gelöst. Die erfindungsgemässe palmare Daumen- und Daumensattelgelenk-Schiene zeichnet sich dadurch aus, dass der erste Fixierungsstreifen vom Handkantenteil über den Handrücken zum ersten Schienenteil verläuft, und der zweite Fixierungsstreifen vom Handkantenteil ausgehend zwischen Daumen und Zeigefinger der geschienten Hand hindurch zum ersten Schienenteil geführt ist.

Auf diese Weise werden die vier Finger der Hand und der Daumen jeweils für sich an der aus dem ersten Schienenteil und dem Handkantenteil zusammengesetzten Schienen-Grundelement mit minimalem Schienenaufwand wirksam fixiert, so dass eine Bewegung beider Handbereiche relativ zueinander unter Einsatz des Daumen-Sattelgelenks sicher unterbunden wird.

Eine Ausgestaltung der erfindungsgemässen palmaren Daumen- und Daumensattelgelenk-Schiene ist dadurch gekennzeichnet, dass an dem ersten Schienenteil eine Fixierungslasche angeordnet ist, welche um den im ersten Schienenteil liegenden Daumen herum gelegt und mit dem freien Ende am ersten Schienenteil fixiert werden kann, damit die Flexion des Daumengrundgelenks unterbunden werden kann.

Eine andere Ausgestaltung der palmaren Daumen- und Daumensattelgelenk-Schiene zeichnet sich dadurch aus, dass der Handkantenteil aus demselben Material besteht wie der erste Schienenteil und eine Fortsetzung des ersten Schienenteils bildet.

Eine weitere Ausgestaltung ist dadurch gekennzeichnet, dass der Handkantenteil mit einer Fixierungslasche verbunden ist, welche sich bei angelegter Schiene über den Handrücken der geschienten Hand erstreckt, und dass der zweite Fixierungsstreifen mit dem einen Ende an der Fixierungslasche befestigt ist.

Vorzugsweise besteht die Fixierungslasche dabei aus demselben Material wie der Handkantenteil und bildet eine Fortsetzung des Handkantenteils.

Eine andere Ausgestaltung der Erfindung zeichnet sich dadurch aus, dass der erste Fixierungsstreifen mit dem einen Ende am Handkantenteil und mit dem anderen Ende am ersten Schienenteil befestigt ist.

Insbesondere ist der erste Fixierungsstreifen an beiden Enden lösbar befestigt, und die Befestigung erfolgt nach Art eines Klettverschlusses.

Vorzugsweise erfolgt auch die lösbare Befestigung des zweiten Fixierungsstreifens an der palmaren Daumen- und Daumensattelgelenk-Schiene nach Art eines Klettverschlusses.

Die leichte Anpassbarkeit und hohe Bequemlichkeit beim Tragen der Schiene lässt sich insbesondere dadurch erreichen, dass die in sich steifen, jedoch mit der Hand plastisch verformbaren Elemente der palmaren Daumen- und Daumensattelgelenk-Schiene aus einem Material bestehen, welches als Kern ein quer gewelltes Aluminiumblech enthält. Ein solches Schienenmaterial ist beispielsweise aus der Druckschrift WO 97/22312 bekannt, auf die wegen der Materialauswahl und Abmessungen des gewellten Bleches ausdrücklich verwiesen wird.

Die Erfindung soll nachfolgend anhand eines Ausführungsbeispiels im Zusammenhang mit den Figuren näher erläutert werden. Es zeigen:
- Fig. 1: in seitlicher Ansicht die Anwendung einer palmaren Daumen- und Daumensattelgelenk-Schiene gemäss einem Ausführungsbeispiel der Erfindung mit zusätzlichem Sattelgelenk-Fixierungsstreifen zum Fixieren des Daumen-Sattelgelenks;
- Fig. 2: die geschiente Hand aus Fig. 1 in der Ansicht schräg von oben;
- Fig. 3: die geschiente Hand aus Fig. 1 in der Ansicht von unten; und
- Fig. 4: die Konfiguration der von der Hand abgenommenen Schiene aus Fig. 1.

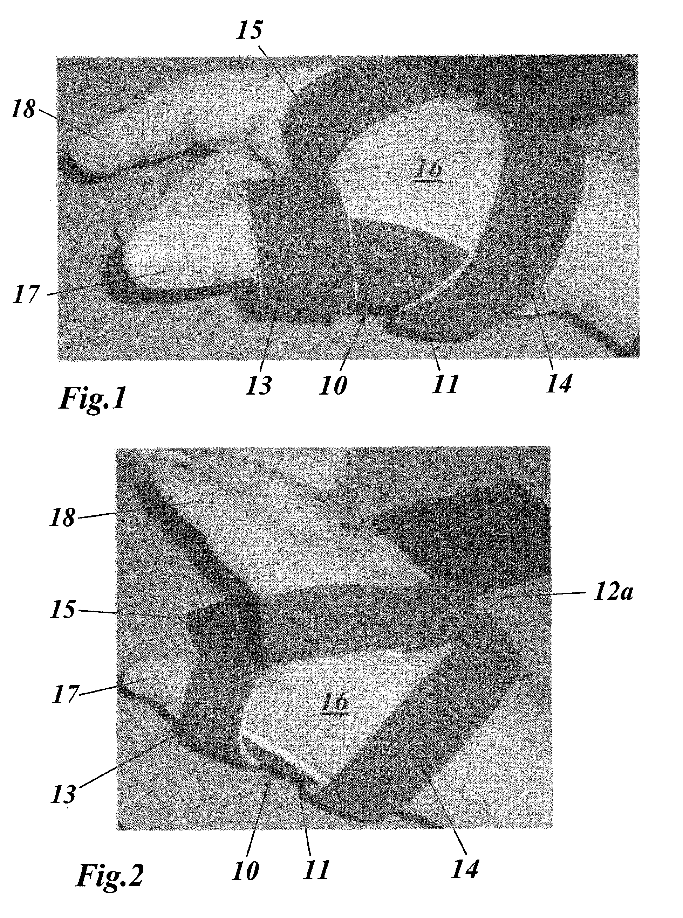

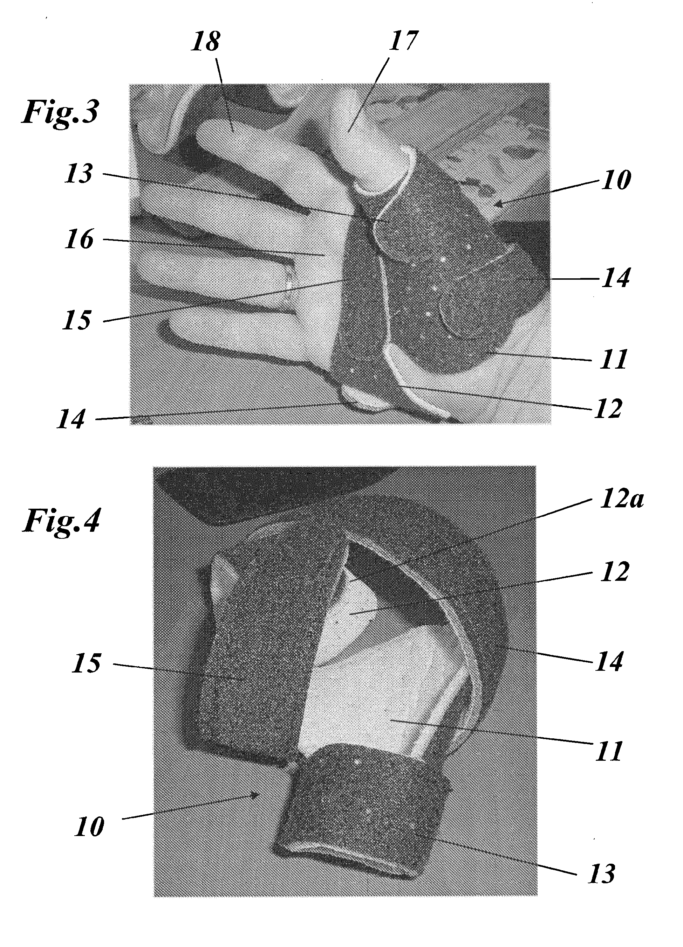

In den Fig. 1, 2 und 3 ist ein Ausführungsbeispiel einer palmaren Daumen- und Daumensattelgelenk-Schiene im Einsatz an einer Hand aus verschiedenen Blickwinkeln dargestellt. Fig. 4 zeigt dieselbe Schiene nach der Abnahme von der Hand.

Grundelement der in den Figuren gezeigten palmaren Daumen- und Daumensattelgelenk-Schiene 10 ist ein in sich steifer, jedoch mit der Hand plastisch verformbarer erster Schienenteil 11, in den der Daumen 17 mit seinem Metacarpalbereich von oben eingelegt wird. Aufgrund seiner ohne weitere Hilfsmittel möglichen plastischen Verformbarkeit kann der erste Schienenteil 11 an die jeweilige Form des Daumens 17 angepasst werden, um eine bequeme und sichere Stützung des Daumens 17 im Metacarpalbereich zu erreichen. Die intrinsische Steifigkeit und plastische Verformbarkeit wird vorzugsweise durch Verwendung eines Schienenmaterials erzielt, das in der o.g. WO 97/22312 eingehend beschrieben ist und dessen mechanische Eigenschaften auf den besonderen Eigenschaften eines quer gewellten Aluminiumbleches beruhen.

Wie in Fig. 4 erkennbar ist, kann das Material des ersten Schienenteils 11 mit Vorteil beidseitig mit (unterschiedlichen) Abdeckschichten versehen sein, wobei die der Haut zugewandte Seite zur weiteren Verbesserung des Tragekomforts auch bei längerem Einsatz beispielsweise mit einer hautsympathischen, atmungsaktiven Polsterung ausgestattet ist, während auf der Aussenseite mit Vorteil ein Deckmaterial verwendet wird, das (durch Ausbildung von flächig verteilten Schlingen oder Haken) Teil eines Klettverschlusses sein kann. Dieses Deckmaterial kann sich insbesondere über die gesamte Fläche erstrecken, um die Befestigung von zusätzlichen Elementen per Klettverschluss an beliebigen Stellen der Schiene zu ermöglichen. Darüber hinaus kann das Schienenmaterial und ggf. auch die Fixierungselemente - wie in den Figuren erkennbar - mit einer Perforation versehen sein, durch die zusätzlich Luft an die geschienten Bereiche gelangen bzw. Hautausdünstungen nach aussen abgegeben werden können.

Zu einer ersten Fixierung des Daumens 17 im angeformten ersten Schienenteil 11 ist an dem ersten Schienenteil 11 quer zur Daumenlängsrichtung eine Fixierungslasche 13 angeordnet, welche um den im Schienenteil 11 liegenden Daumen 17 herum gelegt und mit dem freien Ende am ersten Schienenteil 11 fixiert werden kann (siehe vor allem Fig. 1). Die Fixierungslasche 13 kann beispielsweise aus einem üblichen flexiblen Bandmaterial bestehen. Vorzugsweise ist die Fixierungslasche 13 am ersten Schienenteil 11 mittels eines Klettverschlusses fixierbar, der die Eigenschaften der äusseren Abdeckschicht des ersten Schienenteils 11 ausnutzt. Da die Fixierungslasche 13 gegenüber dem ersten Schienenteil 11 von vergleichsweise geringer Breite ist, hat das Endglied des Daumens 17 im ersten Schienenteil (11) trotz dieser Fixierung ausreichend Bewegungsfreiheit.

Als weiteres Element umfasst die palmare Daumen- und Daumensattelgelenk-Schiene 10 als zweiten Schienenteil einen in Fig. 3 und 4 gut sichtbaren Handkantenteil 12, der mit dem ersten Schienenteil 11 in einer einstückigen Einheit verbunden ist und bei angelegter Schiene die Handkante der geschienten Hand 16 seitlich umfasst. Erster Schienenteil 11 und Handkantenteil 12 bilden zusammen eine Auflagefläche für die Hand 16 und fixieren so in einem ersten Schritt die Lage des geschienten Daumens 17 relativ zur Hand 16. Der Handkantenteil 12 besteht aus demselben Material wie der Schienenteil 11 und ist daher in gleicher Weise in sich steif und mit der Hand plastisch verformbar, so dass er leicht und dauerhaft an die jeweilige Handform angepasst werden kann.

Der Handkantenteil 12 geht über in eine weitere Fixierungslasche 12a, welche sich bei angelegter Schiene über den Handrücken der geschienten Hand 16 erstreckt (siehe insbesondere Fig. 2) und aus demselben in sich steifen, jedoch mit der Hand plastisch verformbaren Material besteht, wie der Handkantenteil 12. Wie in Fig. 2 und Fig. 3 gut zu erkennen ist, ist zur Fixierung der vier Finger der restlichen Hand an der palmaren Daumen- und Daumensattelgelenk-Schiene 10 ein in sich steifer, jedoch mit der Hand plastisch verformbarer Fixierungsstreifen 15 angebracht, der zwischen Daumen 17 und Zeigefinger 18 der geschienten Hand 16 hindurch geführt wird und an der palmaren Daumen- und Daumensattelgelenk-Schiene 10 lösbar befestigt ist. Die lösbare Befestigung des Fixierungsstreifens 15 an der palmaren Daumen- und Daumensattelgelenk-Schiene 10 erfolgt vorzugsweise wiederum nach Art eines Klettverschlusses, wobei das eine Ende mit Vorteil an der Fixierungslasche 12a befestigt wird (Fig. 2).

Ein weiteres Grundelement der palmaren Daumen- und Daumensattelgelenk-Schiene 10 neben dem ersten Schienenteil 11 und dem Handkantenteil 12 ist ein in sich steifer, jedoch mit der Hand plastisch verformbarer, über den Handrücken der geschienten Hand 16 geführter Fixierungsstreifen 14 (siehe insbesondere Fig. 2). Der Fixierungsstreifen 14 ist als separates Teil ausgebildet und mit beiden Enden am ersten Schienenteil 11 bzw. Handkantenteil 12 lösbar befestigt, wobei die Befestigung wiederum nach Art eines Klettverschlusses erfolgt. Da der Fixierungsstreifen 14 - anders als ein biegsames Befestigungsband der üblichen Art - selbst aus Schienungsmaterial besteht, kann er in gleicher Weise an die jeweilige Handform angepasst werden und trägt wesentlich zur Fixierung bei. Insbesondere fixiert er den im ersten Schienenteil 11 ruhenden Daumen 17 relativ zur übrigen Hand. Durch die beiden Fixierungsstreifen 14 und 15 werden so - für sich getrennt - die vier Finger der Hand und der Daumen jeweils in der aus den Teilen 11 und 12 bestehenden Schiene fixiert, so dass jede mögliche Bewegung im Daumengrundgelenk und im Daumen-Sattelgelenk, insbesondere auch die Bewegung Opposition und Reposition, sicher und auf bequeme Weise unterbunden ist.

Insgesamt ergibt sich mit der Erfindung eine palmare Daumen- und Daumensattelgelenk-Schiene, die eine lokale und gezielte Immobilisierung des Daumen-Sattelgelenks, insbesondere auch bei der Rhizarthrose, ermöglicht, einfach in der Anwendung ist, ohne zusätzliche Hilfsmittel auch im Notfall eingesetzt werden kann und sich durch einen hohen Tragekomfort auszeichnet.

Es ist aber auch denkbar, die ersten und zweiten Fixierungsstreifen 14 und 15 nicht aus einem in sich steifen, jedoch mit der Hand plastisch verformbaren Material auszuführen, sondern als Textilbänder auszubilden. In diesem Fall fixiert die Schiene 10 noch immer das Daumengrundgelenk und kann mit Vorteil als palmare Daumenschiene eingesetzt werden.

### Bezugszeichenliste

- 10: palmare Daumen- und Daumensattelgelenk-Schiene
- 11: erster Schienenteil
- 12: Handkantenteil (zweiter Schienenteil)
- 12a: Fixierungslasche
- 13: Fixierungslasche
- 14,15: Fixierungsstreifen
- 16: Hand
- 17: Daumen
- 18: Zeigefinger

## Patentansprüche

1. Palmare Daumen- und Daumensattelgelenk-Schiene (10), umfassend einen ersten Schienenteil (11) zur Aufnahme des Daumens (17), sowie Mittel (14, 15) zum Befestigen des ersten Schienenteils (11) an der Hand (16), wobei der erste Schienenteil (11) als in sich steifer, jedoch mit der Hand plastisch verformbarer, nach oben offener Schalenteil ausgebildet ist, in welchen der Daumen (17) hineingelegt werden kann, wobei der erste Schienenteil (11) fest mit einem in sich steifen, jedoch mit der Hand plastisch verformbaren Handkantenteil (12) verbunden ist, welcher bei angelegter Schiene die Handkante der geschienten Hand (16) umfasst, und wobei die Befestigungsmittel in sich steife, jedoch mit der Hand plastisch verformbare, erste und zweite Fixierungsstreifen (14, 15) umfassen, **dadurch gekennzeichnet, dass** der erste Fixierungsstreifen (14) vom Handkantenteil (12) über den Handrücken zum ersten Schienenteil (11) verläuft, und der zweite Fixierungsstreifen (15) vom Handkantenteil (12) ausgehend zwischen Daumen (17) und Zeigefinger (18) der geschienten Hand (16) hindurch zum ersten Schienenteil (11) geführt ist.

2. Palmare Daumen- und Daumensattelgelenk-Schiene nach Anspruch 1, **dadurch gekennzeichnet, dass** an dem ersten Schienenteil (11) eine Fixierungslasche (13) angeordnet ist, welche um den im ersten Schienenteil (11) liegenden Daumen (17) herum gelegt und mit dem freien Ende am ersten Schienenteil (11) fixiert werden kann.

3. Palmare Daumen- und Daumensattelgelenk-Schiene nach Anspruch 1 oder 2, **dadurch gekennzeichnet, dass** der Handkantenteil (12) aus demselben Material besteht wie der erste Schienenteil (11) und eine Fortsetzung des ersten Schienenteils (11) bildet.

4. Palmare Daumen- und Daumensattelgelenk-Schiene nach einem der Ansprüche 1 bis 3, **dadurch gekennzeichnet, dass** der Handkantenteil (12) mit einer Fixierungslasche (12a) verbunden ist, welche sich bei angelegter Schiene über den Handrücken der geschienten Hand (16) erstreckt, und dass der zweite Fixierungsstreifen (15) mit dem einen Ende an der Fixierungslasche (12a) befestigt ist.

5. Palmare Daumen- und Daumensattelgelenk-Schiene nach Anspruch 4, **dadurch gekennzeichnet, dass** die Fixierungslasche (12a) aus demselben Material besteht wie der Handkantenteil (12) und eine Fortsetzung des Handkantenteils (12) bildet.

6. Palmare Daumen- und Daumensattelgelenk-Schiene nach einem der Ansprüche 1 bis 5, **dadurch gekennzeichnet, dass** der erste Fixierungsstreifen (14) mit dem einen Ende am Handkantenteil (12) und mit dem anderen Ende am ersten Schienenteil (11) befestigt ist.

7. Palmare Daumen- und Daumensattelgelenk-Schiene nach Anspruch 6, **dadurch gekennzeichnet, dass** der erste Fixierungsstreifen (14) an beiden Enden lösbar befestigt ist, und dass die Befestigung nach Art eines Klettverschlusses erfolgt.

8. Palmare Daumen- und Daumensattelgelenk-Schiene nach einem der Ansprüche 1 bis 7, **dadurch gekennzeichnet, dass** die lösbare Befestigung des zweiten Fixierungsstreifens (15) an der palmaren Daumen- und Daumensattelgelenk-Schiene (10) nach Art eines Klettverschlusses erfolgt.

9. Palmare Daumen- und Daumensattelgelenk-Schiene nach einem der Ansprüche 1 bis 8, **dadurch gekennzeichnet, dass** die in sich steifen, jedoch mit der Hand plastisch verformbaren Elemente (11,..,15) der palmaren Daumen- und Daumensattelgelenk-Schiene (10) aus einem Material bestehen, welches als Kern ein gewelltes Aluminiumblech enthält.

## Claims

1. Palmar splint for the thumb and carpometacarpal joint of the thumb (10), comprising a first splint part (11) for receiving the thumb (17), and means (14, 15) for attaching the first splint part (11) to the hand (16), wherein the first splint part (11) is designed as shell part which is open at the top and intrinsically rigid but plastically deformable by the hand, into which the thumb (17) can be placed, wherein the first splint part (11) is firmly connected to a part for the side of the hand (12) which is intrinsically rigid but plastically deformable by the hand and which encompasses the side of the hand of the splinted hand (16) when the splint is applied, and wherein the fasteners comprise first and second immobilizing straps (14, 15) which are intrinsically rigid but plastically deformable by the hand, **characterized in that** the first immobilizing strap (14) extends from the part for the side of the hand (12) over the back of the hand to the first splint part (11), and the second immobilizing strap (15) is guided from the part for the side of the hand (12), starting between and proceeding through thumb (17) and index finger (18) of the splinted hand (16), to the first splint part (11).

2. Palmar splint for the thumb and carpometacarpal joint of the thumb according to claim 1, **characterized in that** an immobilizing flap (13) is arranged on the first splint part (11) which can be placed around the thumb (17) positioned in the first splint part (11) and immobilized with the free end on the first splint part (11).

3. Palmar splint for the thumb and carpometacarpal joint of the thumb according to claim 1 or 2, **characterized in that** the part for the side of the hand (12) is made of the same material as the first splint part (11) and forms a continuation of the first splint part (11).

4. Palmar splint for the thumb and carpometacarpal joint of the thumb according to one of the claims 1 to 3, **characterized in that** the part for the side of the hand (12) is connected to an immobilizing flap (12a) which extends over the back of the hand of the splinted hand (16) when the splint is applied, and that the second immobilizing strap (15) is attached with one end to the immobilizing flap (12a).

5. Palmar splint for the thumb and carpometacarpal joint of the thumb according to claim 4, **characterized in that** the immobilizing flap (12a) is made of the same material as the part for the side of the hand (12) and forms a continuation of the part for the side of the hand (12).

6. Palmar splint for the thumb and carpometacarpal joint of the thumb according to one of the claims 1 to 5, **characterized in that** the first immobilizing strap (14) is attached with one end to the part for the side of the hand (12) and with the other end attached to the first splint part (11).

7. Palmar splint for the thumb and carpometacarpal joint of the thumb according to claim 6, **characterized in that** the first immobilizing strap (14) is detachably fastened on both ends, and that it is fastened with a hook and loop-style fastener.

8. Palmar splint for the thumb and carpometacarpal joint of the thumb according to one of the claims 1 to 7, **characterized in that** the second immobilizing strap (15) is detachably fastened to the palmar splint for the thumb and carpometacarpal joint of the thumb (10) with a hook and loop-style fastener.

9. Palmar splint for the thumb and carpometacarpal joint of the thumb according to one of the claims 1 to 8, **characterized in that** the elements (11,.., 15) of the palmar splint for the thumb and carpometacarpal joint of the thumb (10), which are intrinsically rigid but plastically deformable by the hand, are made of a material which contains corrugated aluminum sheet metal as its core.

## Revendications

1. Attelle palmaire du pouce et de l'articulation trapézo-métacarpienne du pouce (10), comprenant une première partie d'attelle (11) destinée à recevoir le pouce (17), ainsi que des moyens (14, 15) destinée à fixer la première partie d'attelle (11) sur la main (16), dans laquelle la première partie d'attelle (11) est conçue comme une partie de coque ouverte vers le haut, rigide en soi mais plastiquement déformable à la main, dans laquelle peut être disposé le pouce (17), la première partie d'attelle (11) étant fermement reliée à une partie de tranche de main (12) rigide en soi mais déformable plastiquement à la main, laquelle entoure le tranchant de la main mise en attelle (16) lorsque l'attelle est appliquée, et les moyens de fixation comprenant des premières et deuxièmes bandes de fixation (14, 15) rigides en soi mais plastiquement déformables à la main, **caractérisée en ce que** la première bande de fixation (14) s'étend de la partie de tranche de main (12) à la première partie d'attelle (11) en passant par le dos de la main, et la deuxième bande de fixation (15) est guidée entre le pouce (17) et l'index (18) de la main mise en attelle (16) en partant de la partie de tranche de main (12) jusqu'à la première partie d'attelle (11).

2. Attelle palmaire du pouce et de l'articulation trapézo-métacarpienne du pouce selon la revendication 1, **caractérisée en ce qu'**une languette de fixation (13) est disposée sur la première partie d'attelle (11), laquelle est placée autour du pouce (17) situé dans la première partie d'attelle (11) et peut être fixée par son extrémité latérale sur la première partie d'attelle (11).

3. Attelle palmaire du pouce et de l'articulation trapézo-métacarpienne du pouce selon la revendication 1 ou 2, **caractérisée en ce que** la partie de tranche de main (12) est constituée du même matériau que la première partie d'attelle (11) et forme un prolongement de la première partie d'attelle (11).

4. Attelle palmaire du pouce et de l'articulation trapézo-métacarpienne du pouce selon l'une des revendications 1 à 3, **caractérisée en ce que** la partie de tranche de main (12) est reliée à une languette de fixation (12a) s'étendant sur le dos de la main mise en attelle (16) lorsque l'attelle est appliquée, et **en ce que** la deuxième bande de fixation (15) est fixée à la languette de fixation (12a) par l'une des extrémités.

5. Attelle palmaire du pouce et de l'articulation trapézo-métacarpienne du pouce selon la revendication 4, **caractérisée en ce que** la languette de fixation (12a) est constituée du même matériau que la partie de tranche de main (12) et forme un prolongement de la partie de tranche de main (12).

6. Attelle palmaire du pouce et de l'articulation trapézo-métacarpienne du pouce selon l'une des revendications 1 à 5, **caractérisée en ce que** la première bande de fixation (14) est fixée par l'une de ses extrémités à la partie de tranche de main (12) et par l'autre de ses extrémités à la première partie d'attelle (11).

7. Attelle palmaire du pouce et de l'articulation trapézo-métacarpienne du pouce selon la revendication 6, **caractérisée en ce que** la première bande de fixation (14) est fixée de façon détachable par les deux extrémités, et **en ce que** la fixation est réalisée à la manière d'un scratch.

8. Attelle palmaire du pouce et de l'articulation trapézo-métacarpienne du pouce selon l'une des revendications 1 à 7, **caractérisée en ce que** la fixation détachable de la deuxième bande de fixation (15) sur l'attelle palmaire du pouce et de l'articulation trapézo-métacarpienne du pouce (10) est réalisée à la manière d'un scratch.

9. Attelle palmaire du pouce et de l'articulation trapézo-métacarpienne du pouce selon l'une des revendications 1 à 8, **caractérisée en ce que** les éléments (11, ..., 15) rigides en soi mais plastiquement déformables à la main de l'attelle palmaire du pouce et de l'articulation trapézo-métacarpienne du pouce (10) sont constitués d'un matériau contenant une tôle d'aluminium ondulée comme noyau.
